# EUROPÄISCHE PATENTSCHRIFT

(11) **EP 0 668 874 B1**
(45) Veröffentlichungstag und Bekanntmachung des Hinweises auf die Patenterteilung: **26.08.1998**
(21) Anmeldenummer: 92910746.4
(22) Anmeldetag: 22.05.1992
(51) Int. Cl.: C07K 7/23, A61K 38/22, C07D 233/61, C07D 249/08, C07D 295/145, C07C 227/22

(54) **LHRH-ANTAGONISTEN UND ZWISCHENPRODUKTE**
LHRH ANTAGONISTS AND INTERMEDIATE PRODUCTS
ANTAGONISTES DE LA LHRH ET PRODUITS INTERMEDIAIRES

(30) Priorität: 24.05.1991 DE 4117507
(43) Veröffentlichungstag der Anmeldung: 30.08.1995
(73) Patentinhaber: SCHERING AKTIENGESELLSCHAFT, 13353 Berlin (DE)
(72) Erfinder: THAMM, Paul, Dr., D-82024 Taufkirchen (DE); LOBBIA, Alessandro, Dr., D-12099 Berlin (DE); BRUMBY, Thomas, Dr., D-10827 Berlin (DE); MULZER, Johann, Prof. Dr., D-13467 Berlin (DE); SCHRÖDER, Fridtjof, Dr., D-14195 Berlin (DE); HABENICHT, Ursula, Dr., D-14167 Berlin (DE)
(86) Internationale Anmeldenummer: DE9200427
(87) Internationale Veröffentlichungsnummer: WO9220711

(56) Entgegenhaltungen:
- EP-A- 0 277 829
- EP-A- 0 299 402
- EP-A- 0 410 260
- WO-A-89/01944
- Science in China, Band 34, Nr. 2, Februar 1991; L. Ke-Liang et al.: "Synthesis and bioactivities of new LHRH antagonists containing novel unnatural amino acids at position five", Seiten 201-208, siehe den ganzen Artikel
- Synthesis, Nr. 3, März 1975; C.F. Lane: "Sodium cyanoborohydride - a highly selective reducing agent for organic functional groups", Seiten 135-146, siehe Seite 144, Spalte 1 (in der Anmeldung erwähnt)
- Journal of the Chemical Society, 1957, part IV, Nomenclature Report, and Indexes, (London, GB) B.C. Barrass et al.: "The formation of cyclic lactams from derivatives of basic amino-acids", Seiten 4830-4834, siehe Seite 4831, "Experimental" (in der Anmeldung erwähnt)

## Beschreibung

Das Luteneizing Hormone Releasing Hormone (LHRH) pyroGlu¹-His²-Trp³-Ser⁴-Tyr⁵-Gly⁶-Leu⁷-Arg⁸-Pro⁹-Gly¹⁰-NH₂, wird bei Säugern im Hypothalamus produziert. Es stimuliert in der Hypophyse die Ausschüttung von Luteinizing Hormone (LH) und Follicle Stimulating Hormone (FSH). Diese wiederum kontrollieren die Produktion der Androgene und Oestrogene in den Geschlechtsorganen.

Durch Verabreichung von Einzelgaben von LHRH oder synthetischer Agonisten läßt sich eine vermehrte Produktion von Steroidhormonen (z.B. Testosteron) erreichen. Chronische Gabe führt hingegen zu einer Senkung der Hormonproduktion. Dieser Effekt wird seit einiger Zeit zur Behandlung hormonabhängiger Tumore (Prostata CA) verwendet.

Begleiteffekt dieser Therapie ist die initiale Stimulation der zu unterdrückenden Hormone. Dieser Effekt, der zu einem vorübergehenden Tumorwachstum (tumour flare up) bei hormonabhängigen Tumoren führt, kann durch Verwendung von LHRH-Antagonisten vermieden werden. Karten et al. (Endocrine Rev. 7,44,1988) und Dutta (Drugs of the Future 13,761,1988) haben die Entwicklung der LHRH-Antagonisten beschrieben.

Wirksame LHRH-Antagonisten, die basische Aminosäuren in den Positionen 6 und 8 haben, setzen jedoch unerwünscht hohe Mengen an Histamin frei. Verschiedene Anstrengungen sind unternommen worden, um die Histaminausschüttung zu reduzieren. Die europäische Publikation EP-OS 097 031 beschreibt Arginin-Derivate in der Position 6. Die europäische Publikation EP-OS 0 277 829 stellt basische Aminosäurederivate in den Positionen 6 und 8 dar. Die europäische Publikation EP-OS 0 299 402 offenbart die Kombination von Citrullin in Position 6 mit Arginin in Position 8, wobei recht wenig Histamin freigesetzt wird.

Es wurde nun überraschend gefunden, daß durch Austausch der Positionen 6 und 8 mit Lysinderivaten, die am Omega-Stickstoff substituiert sind, eine Wirkungssteigerung beziehungsweise Senkung der Histaminfreisetzung erzielt werden kann.

Erfindungsgemäß werden Peptid-Verbindungen der Formel I beansprucht, 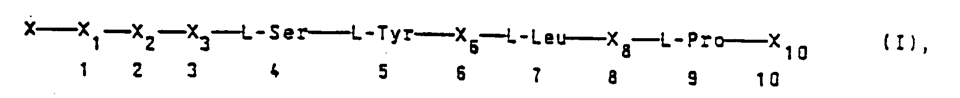 worin
X für eine Naphthoyl-, Naphthylacetyl-, Naphthylpropionyl-, Benzoylgruppe oder eine Acylgruppe mit 1-7 Kohlenstoff-Atomen steht,
X₁ für D-(1)-Nal, D-(2)-Nal, D-Phe, D-(4-Y)-Phe, D-(3)-Qal oder eine Direktbindung steht,
wobei Y eine F-, Br- oder Cl-Gruppe ist,
X₂ für D-Phe, D-(4-Y)-Phe oder eine Direktbindung steht,
wobei Y die zuvor genannte Bedeutung besitzt,
X₃ für D-Trp, D-Phe, D-(4-Y)-Phe, D-(3)-Pal, D-(2)-Nal oder eine Direktbindung steht.
wobei Y die bereits für X₁ genannte Bedeutung besitzt,
X₆ für D-Cit, D-Hci, D-Orn, D-Lys oder D-Neu steht,
X₈ für L-Orn, L-Arg, L-Lys oder L-Neu steht, wobei wenigstens einer der Reste X₆ und X₈ ein Neu ist,
und
X₁₀ für D-Ala-NH₂, Gly-NH₂, Azaglycin, -NHEt oder -NH(CO)NH₂ steht, wobei Neu für einen Rest der Formel IX oder IV 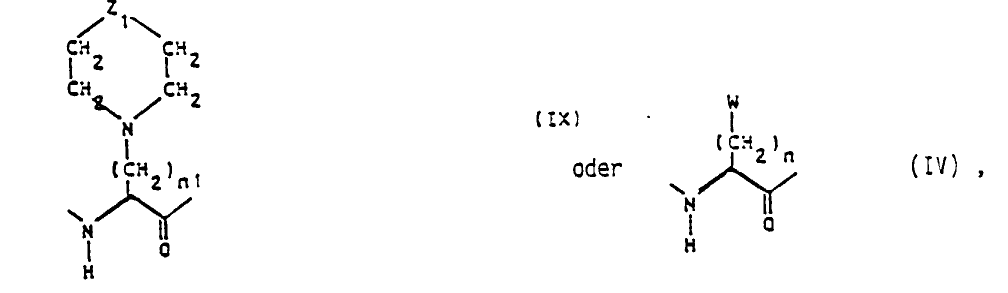 steht, worin Z₁ für eine Gruppe 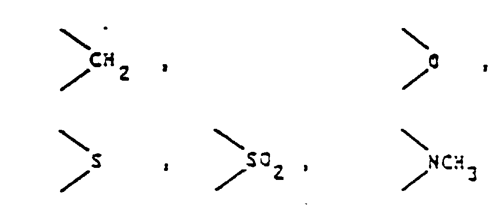 oder eine Direktbindung,
   n für 1 bis 8,
   n₁ für 3 bis 6 stehen und
   W einen der Reste 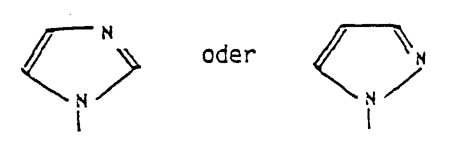 bedeutet.

Der Text umfaßt einige Abkürzungen, deren Bedeutung im weiteren dargelegt wird. Dabei werden die von der IUPAC-IUB Kommission für biochemische Nomenklatur festgelegten Regeln befolgt (Biochemistry 11: 1726 (1972 und Biochem. J. 219: 345 (1984)). Zusätzlich werden folgende Abkürzungen und deren Kombinationen verwendet:

| | | | |
|---|---|---|---|
| Ape | 2-Amino-pentansäure | (1)-Nal | 3-(Naphth-1-yl)-alanin |
| Ahx | 2-Amino-hexansäure | (2)-Nal | 3-(Naphth-2-yl)-alanin |
| Ahp | 2-Amino-heptansäure | (3)-Pal | 3-(3-Pyridyl)-alanin |
| Aoc | 2-Amino-octansaure | (3)-Qal | 3-(Chinol-3-yl)-Alanin |
| Ano | 2-Amino-nonansäure | Hci | Homocitrullin |
| Mor | Morpholin-4-yl- | 1Im | Imidazol-1-yl- |
| Pip | Piperidin-1-yl- | 4Tr | 1,3,4-Triazol-4-yl- |
| Pyr | Pyrrolidin-1-yl- | 1Tr | 1,3,4-Triazol-1-yl- |
| Tht | Tetrahydro-1,4-thiazin-4-yl- | 1Py | Pyrazol-1-yl- |
| Mpz | 4-Methyl-piperazin-1-yl- | Cpz | 4-Carbamoyl-piperazin-1-yl- |
| Pon | 4-Piperidon-1-yl- | Cpa | 4-Chlor-phenylalanin |
| Hpi | 4-Hydroxy-piperidin-1-yl- | | |
| Aps | 4-Aza-pentamethylensulfon-4-yl- | | |

So bedeutet z.B. Aoc(Mor) = 6-Morpholin-4-yl-2-amino-octansäure, Ape(Pip) = 5-Piperidin-4-yl-2-amino-pentansäure und Ahx(1Im) = 6-(Imidazol-1-yl)-2aminohexansäure.

Die Peptide werden in einer abgekürzten Form repräsentiert, wobei nur die im Vergleich zum LHRH veränderten Aminosäuren und ihre Position aufgeführt sind. So wird z.B.
pyroGlu¹-His²-Trp³-Ser⁴-Tyr⁵-D-Nal⁶-Leu⁷-Arg⁸-Pro⁹-D-Ala¹⁰-NH₂ zu [D-Nal⁶, D-Ala¹⁰]LHRH

Die im Text erwähnten Alkylgruppen sind geradlinig oder verzweigt und bedeuten Methyl, Ethyl, Propyl, Isopropyl, n-Butyl, sek.-Butyl, Isobutyl, tert.-Butyl, Pentyl, Isopentyl, Neopentyl, Hexyl, Isohexyl, Heptyl, Isoheptyl.

Die Vorteile der erfindungsgemäßen Peptid-Verbindungen bestehen darin, daß die pharmakologische Wirkung der Peptid-Verbindungen sehr hoch ist und die Nebenwirkungen in Form der Histaminfreisetzung gering gehalten werden.

Bevorzugt sind Peptid-Verbindungen, bei denen Neu für eine Gruppe der Formel X steht, 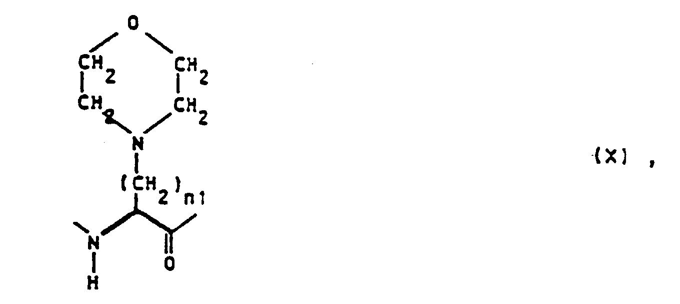 worin n₁ für 3 bis 6 steht.

Mohr bevorzugt sind Peptid-Verbindungen, bei denen Neu für den Rest 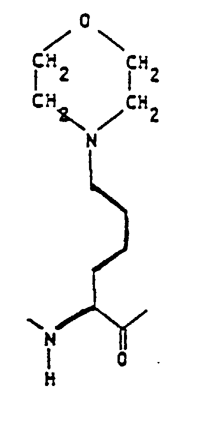 steht.

Am meisten bevorzugt sind Peptid-Verbindungen, bei denen Neu für X₆ oder X₈ steht.

Eine weitere vorteilhafte Ausführungsform besteht darin, daß Neu für die Formel XIII steht

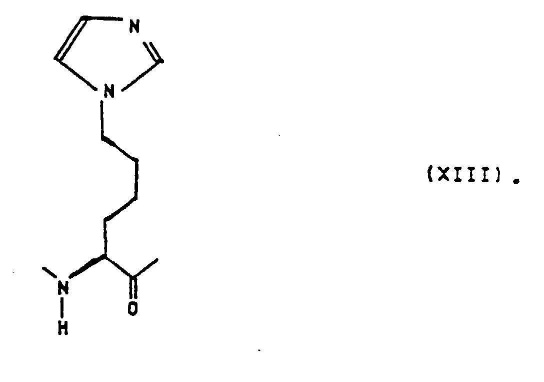

Auch sind Peptid-Verbindungen bevorzugt, in denen
X für eine Ethanoyl-Gruppe,
X₁ für D-Nal,
X₂ für D-Cpa,
X₃ für D-Pal und
X₁₀ für D-Ala-NH₂ steht.

Die am meisten bevorzugte Peptid-Verbindung ist Ac-D-Nal-D-Cpa-D-Pal-Ser-Tyr-D-Cit-Leu-Ahx(Mor)-Pro-D-Ala-NH₂

### Anwendung der Peptid-Verbindungen:

a) Die Erfindung umfaßt Arzneimittel enthaltend eine oder mehrere Verbindungen der Formel I und übliche Hilfs- und Trägerstoffe. Ebenfalls umfaßt die Erfindung pharmazeutische Zusammensetzungen zur Behandlung einer Krankheit, wobei die Zusammensetzung eine Peptid-Verbindung der Formel I umfaßt und wobei die Zusammensetzung weiterhin ein pharmakologisch annehmbares Salz und/oder einen pharmakologisch annehmbaren Träger umfaßt.

Die erfindungsgemäßen Peptid-Verbindungen, deren Salze und Gemische mit pharmakologisch unbedenklichen Träger- und Zusatzstoffen haben eine effektvolle und langandauernde LHRH - antagonistische Wirkung.

Die Peptid-Verbindungen sollen bei der Behandlung von gutartigen Prostata-Hypertrophieen und Prostatakarzinomen verwendet werden. Daher wird das Testosteron senkende Potential getestet. Hierfür werden von ein- und demselben Antagonisten generell deutlich höhere Dosierungen benötigt als für die Induktion einer Ovulationshemmung. In dem hier verwendeten Testverfahren werden intakte adulte männliche Ratten einmalig mit der zu testenden Substanz subkutan behandelt. Der Effekt auf die Serumtestosteron-Konzentration nach 24 Stunden wird radioimmunologisch (Kit der Firma Biermann) ermittelt.

Die Substanz des Beispiels 13 (Punkt 6.1.) induziert in einem Dosisbereich von 0.5 bis 5 mg/kg Körpergewicht eine Hemmung der Serumtestosteron-Konzentration zwischen 80 und 97 % im Vergleich zur Kontrolle. Auch bei einer Dosis von 0,25 mg/kg Körpergewicht ist nach eine Hemmung von 26 % zu beobachten.

Neben der Absenkung des Testosteronwertes ist die Histaminfreisetzung von Bedeutung. So treten bei keiner der getesteten Dosierungen (0.1 bis 5 mg / kg Körpergewicht subcutan) ödematöse Veränderungen im Gesicht und/oder an den Extremitäten auf, wie dies bei einer Histaminfreisetzung typischerweise der Fall ist. Diesem in vivo Test wird wesentlich mehr Relevanz zugemessen als dem vielfach verwendeten Mastzell-Test. Dennoch zeigt auch der Mastzell-Test, daß bei der Verwendung der Peptid-Verbindung des Beispiels 13 die ED₅₀ der Histaminfreisetzung bei 0.01 mg/ml noch nicht erreicht ist.

Weitere Testverfahren für die Wirksamkeit von LHRH-Antagonisten sind i) die Inhibition der FSH- und LH-Freisetzung in Ratten, die durch LHRH induziert wird. (VILCHEZ - MARTINEZ J.A. et al. (1975) Endocrinology, 96, 1130) und ii) die Inhibition der LH- und FSH-Freisetzung durch verteilte, frühe Hypophysenzell-Kulturen, wie sie im Radioimmun-Test ausgetestet werden (VALE et al. (1972) Endocrinology 91: 562).

Die Wirksamkeit der erfindungsgemäßen Peptid-Verbindungen, die zuvor dargelegt worden ist, führt zu einer Reihe sich daraus ableitenden Verwendungen:
aa) Therapie für gutartige Prostatahypertrophie;
bb) Therapie für Krankheiten, die durch gesteigerte Keimdrüsen-Hormon-Praduktion in beiderlei Geschlechtern hervorgerufen wird, insbesondere Prostatakarzinom,
cc) Behandlung von Endometriose,
dd) Fertilitätskontrolle bei Frauen,
ee) Ovulationsunterdrückung oder Ovulationsverzögerung,
ff) Synchronisation der Ovulation,
hh) Östrusunterdrückung,
ii) Wachstumsförderung in weiblichen Tieren,
kk) Menstruationsinduktion,
ll) früher, in den ersten drei Monaten liegender Abort,
mm) Behandlung von Zysten in der Brust,
nn) Behandlung des polyzystischen Ovariensyndroms (Stein-Leventhal),
oo) Fertilitätskontrolle bei Männern;
pp) funktionelle Kastration bei männlichen Tieren in der Fleischproduktion und
qq) Unterdrückung von Menopausen-Symptomen.

Besonders bevorzugt ist die Behandlung von Prostatakarzinom und von Endometriose.

In der Praxis werden eine wirksame Henge der Peptid-Verbindung gemäß der Formel 1 oder eine wirksame Menge eines Gemisches, das die Peptid-Verbindung gemäß der Formel 1 und Träger und/oder Zusatzstoffe enthält, dem Menschen oder Tier verabreicht, der oder das eine solche Behandlung benötigt. Die Peptid-Verbindung oder das Gemisch können mittels verschiedener Arten verabreicht werden, wobei diese oder dieses oral, intravenös, subkutan, intramuskulär, intravaginal, rektal oder nasal verabreicht werden kann. Die entsprechende Verabreichungsart wird durch die Form der Behandlung und durch die Dosis bestimmt. De nach Verwendung kann eine Depot-Form, ein Implantat oder eine den Wirkstoff langsam freigebende galenische Form verwendet werden.

Bei der Behandlung von Prostatakarzinom beim Menschen (Behandlung mit hohen Dosen) werden tägliche Dosen im von 1 bis 10, vorzugsweise im Bereich von 2 bis 4 mg pro Person verabreicht.

Die genaue Dosis und die Form der Verabreichung hängt im einzelnen von der Peptid-Verbindung gemäß der Formel 1, von der Art der Verabreichung (Weg in die Blutbahn), und von der Art und der Schwere der zu behandelnden Zustände ab.
b) Die Erfindung umfaßt weiterhin eine Verwendung einer Peptid-Verbindung gemäß Formel I zur Anwendung nach einem der Punkte aa) bis qq). Die Erfindung umfaßt ebenfalls ein Verwendung einer Peptid-Verbindung gemäß der Formel I zur Herstellung eines Medikaments für die therapeutische Anwendung nach einem der Punkte aa) bis qq). Ebenfalls betrifft die Erfindung ein Verfahren zur Anwendung nach einem der Punkte aa) bis qq) bei Menschen und Säugetieren, die eine solche Verwendung benötigen, wobei die Verwendung eine Verabreichung einer pharmakologisch sicheren und wirkungsvollen Menge der Peptid-Verbindungen gemäß der Formel I bei Menschen und Säugetieren umfaßt.
c) Die Erfindung umfaßt bevorzugt eine Verwendung einer Peptid-Verbindung gemäß Formel I zur Behandlung von Prostatakarzinom. Die Erfindung umfaßt ebenfalls eine Verwendung einer Peptid-Verbindung gemäß der Formel I zur Herstellung eines Medikaments zur Behandlung von Prostatakarzinom. Ebenfalls betrifft die Erfindung ein Verfahren zur Behandlung von Prostatakarzinom bei Menschen und Säugetieren, die eine solche Behandlung benötigen, wobei die Behandlung eine Verabreichung einer pharmakologisch sicheren und wirkungsvollen Menge der Peptid-Verbindungen gemäß der Formel I bei Menschen und Säugetieren umfaßt..
d) Die Erfindung umfaßt bevorzugt eine Verwendung einer Peptid-Verbindung gemäß Formel I zur Behandlung von Endometriose. Die Erfindung umfaßt ebenfalls eine Verwendung mit einer Peptid-Verbindung gemäß der Formel I zur Herstellung eines Medikaments zur Behandlung von Endometriose. Ebenfalls betrifft die Erfindung ein Verfahren zur Behandlung von Endometriose bei Menschen und Säugetieren, die eine solche Behandlung benötigen, wobei die Behandlung eine Verabreichung einer pharmakologisch sicheren und wirkungsvollen Menge der Peptid-Verbindungen gemäß der Formel I bei Menschen und Säugetieren umfaßt.
e) Die Erfindung umfaßt bevorzugt eine Verwendung einer Peptid-Verbindung gemäß Formel I zur Fertilitätskontrolle. Die Erfindung umfaßt ebenfalls ein Verwendung einer Peptid-Verbindung gemäß der Formel I zur Herstellung eines Medikaments zur Fertilitätskontrolle. Ebenfalls betrifft die Erfindung ein Verfahren zur Fertilitätskontrolle bei Menschen und Säugetieren, die eine solche Behandlung benötigen, wobei die Behandlung eine Verabreichung einer pharmakologisch sicheren und wirkungsvollen Menge der Peptid-Verbindungen gemäß der Formel I bei Menschen und Säugetieren umfaßt.

Die erfindungsgemäßen Peptidverbindungen werden unter Verwendung von bekannten Aminosäurederivaten hergestellt, indem man die Aminosäurederivate in einer homogenen Phase oder nach der Festphasen-Methode kondensiert,
wobei a) das Carboxyl-Ende eines zu koppelnden Aminosäurederivates, deren Aminogruppen und gegebenenfalls funktionellen Gruppen der Seitenkette eine Schutzgruppe tragen, mit dem freien Amino-Ende des zu koppelnden Aminosäurederivates oder des zu koppelnden Peptidfragments in Gegenwart eines Kondensationsreagenzes reagiert,
und b) anschließend die α-Amino-Schutzgrugpe des gekoppelten Aminosäure-derivates abgespalten wird und
gegebenenfalls weitere Aminosäurederivate an die zu synthetisierenden Peptid-Kette nach den zuvor beschriebenen beiden Schritten gekoppelt werden und nach Kopplung der letzten-Aminosäure im Falle der Festphasen-Methode die Peptid-Verbindung von der Festphase abgespalten wird.

Die nachfolgenden Ausführungsbeispiele dienen zur näheren Erläuterung der Herstellung der erfindungsgemäßen Verbindungen.

### Allgemeine Synthese der Peptide

Die Peptide der Erfindung können mittels der Techniken hergestellt werden, die den Fachleuten im Bereich der Peptidsynthese bekannt sind. Eine Zusammenfassung vieler dieser Techniken können bei J.M. STEWART and J.D. YOUNG, San Francisco, 1969, and J. MEIENHOFER, Hormonal Proteins and Peptides, Vol. 2 p 45. Academic Press (New York), 1973 für die Festphasen-Methode and E. SCHRODER and K. Lubke, The Peptides, Vol. 1, Academic Press (New York) 1965 für die Flüssigphasen-Methode nachgelesen werden. Die Schritte der Synthese sind in den EP-OS 0 097 031 beschrieben.

Die allgemeinen Verfahrensschritte aus der europäischen Offenlegungsschrift lassen sich analog auf die Synthese der hier beschriebenen erfindungsgemäßen Peptid-Verbindungen übertragen.

Spezieller dargestellt lassen sich die erfindungsgemäßen Peptide wie folgt herstellen:

Die Peptide werden schrittweise auf einem ca. 0.5 mÄquiv. NH2/g enthaltenden Benzhydrylamin-Harz auf einem ACT-Synthesizer aufgebaut, indem mit FMOC-D-Ala gemäß beschriebenen Verfahren begonnen wurde.

Die Kopplungen werden gemäß Schema A wie folgt durchgeführt:

### SCHEMA A

Reagenz
1. FMOC-Aminosäure (2 bis 3 mmol/g Harz)
2. 4 Äquivalente Hydroxybenzotriazol-Hydrat berechnet auf eingesetzte Aminosäuren
3. 4 Äquivalente BOF-Reagenz
4. 4 Äquivalente Diisopropylethylamin

Als Lösungsmittel wird N,N-Dimethylformamid verwendet. Die Kupplungszeit beträgt ca. 30 Minuten.

Die Deblockierung wird gemäß Schema B durchgeführt:

### SCHEMA B

5. Spülen mit Dimethylformamid (2 mal)
6. 20 % Piperidin in Dimethylformamid, 3 mal in 3 Minuten.
7. Spülen mit Dimethylformamid (2 mal)

Zusammengefaßt wird das FMOC zum Schutz der α-Aminogruppen verwendet. tBu wird als Schutzgruppe für die Hydroxygruppe des Ser und die phenolische Hydroxygruppe des Tyr verwendet. Zum Schutz der Guanido-Funktionen des Arg wird die Mtr-Gruppe verwendet.

Um das geschützte Peptid-Harz zu spalten und zu entschützen, wird es mit Trifluoressigsäure über einen Zeitraum von mindestens einer Stunde behandelt. Die Trifluoressigsäure wird vom ungelösten Harz abgetrennt und im Vakuum zur Trockne eingedampft. Aus dem Rückstand wird durch präparative HPLC nach bekannten Verfahren das gewünschte Peptid in reiner Form isoliert.

### Beispiele

### 1.1. Herstellung von 5-(1-Aza-cycloalk-1-yl)-2-acetamido-2-ethoxycarbonyl-pentansäureethylester (Allgemeine Vorschrift)

Zu 5,0 g (23 mmol) Acetamido-malonester werden zunächst 10 mL Benzol und 1 mmol Natrium-methoxid gegeben. Dann tropft man 23 mmol Acrolein während 30 Min. hinzu, wobei man durch äußere Kühlung mit Eiswasser dafür sorgt, daß die Reaktionstemperatur +35°C nicht überschreitet. Man rührt 60 Min. nach (wobei die Temperatur auf ca. +10°C ansteigt) und stellt mit Essigsäure auf pH 7 (angefeuchtetes Indikatorpapier). Die Reaktionslösung wird im Vakuum eingedampft und der ölige Rückstand in 20 mL Methanol (getrocknet über Molekularsieb, 4 Å) gelöst.

Man kühlt erneut mit Eiswasser und gibt nacheinander 23 mmol Aza-cycloalkan, 46 mmol Natriumacetat, 2,5 g Molekularsieb (4 Å) und zuletzt 46 mmol Natriumcyanoborhydrid hinzu. Nach Beendigung der Gasentwicklung wird 16 Stdn. bei +20°C gerührt. Man stellt mit wäßriger Natriumcarbonat-Lösung auf pH 10, extrahiert dreimal mit je 100 mL Essigsäureethylester, vereinigt die organischen Phasen, extrahiert mit gesättigter Natriumchlorid-Lösung, trocknet über Natriumsulfat und dampft das Filtrat ein.

### 1.2. Herstellung von drei speziellen Pentansäureethylestern

### 1.2.1. Beispiel 1: Herstellung von 5-(Morpholin-4-yl)-2-acetamido-2-ethoxycarbonyl-pentansäureethylester:

Ausgehend von 21,7 g Acetamidomalonester erhält man 34,5 g Rohprodukt, aus dem nach Kieselgel-Chromatographie (Dichlormethan, Methanol 95:5; v/v) 12,8 g Reinprodukt (Öl) erhalten werden.

### 1.2.2. Beispiel 2: Herstellung von 5-(Pyrrolidin-1-yl)-2-acetamido-2ethoxycarbonyl-pentansäureethylester:

Ausgehend von 5,0 g Acatamidomalonester erhält man 6,6 g Rohprodukt, das ohne weitere Reinigung weiter umgesetzt wird.

### 1.2.3. Beispiel 3: Herstellung von 5-(Thiomorpholin-4-yl)-2-acetamido-2ethoxycarbonyl-pentansäureethylester:

Ausgehend von 5,0 g Acetamidomalonester erhält man 7,5 g Rohprodukt, aus dem nach Kieselgel-Chromatographie (Dichlormethan, Methanol 95:5; v/v) das Reinprodukt erhalten werden.

### 2. Herstellung von 6-Brom-2-acetamido-2-ethoxycarbonyl-hexansäureethylester (Beispiel 4)

Das Gemisch aus 86,9 g Acetamido-malonester, 215,9 g 1,4-Dibrombutan, 4 g Triethyl-benzyl-ammonium-chlorid, 82,8 g Kaliumcarbonat und 400 mL Acetonitril wird 24 Stunden zum Rückfluß erhitzt. Die ungelösten Bestandteile werden über Celite abfiltriert, das Filtrat im Vak. eingedampft und der Rückstand dreimal mit je 500 mL Wasser im Vak. eingedampft. Der Rückstand wird mit 500 mL Diethylether digeriert und über Nacht bei +5°C stehen gelassen. Das Filtrat vom Ungelösten wird im Vak. eingedampft. Der Rückstand wird durch Kieselgel-Chromatographie (tert.Butyl-methylether, Hexan; 7:3; v/v) gereinigt. Man erhält 64 g Reinprodukt.
Schmp. 61-62°C.

### 3.1. Herstellung von 6-(1-Aza-cycloalk-1-yl)-2-acetamido-2-ethoxycarbonylhexansäureethylester (Allgemeine Vorschrift)

352 mg 6-Brom-2-acetamido-2-ethoxycarbonyl-hexansäureethylester (vergleiche Beispiel 4) werden zur Lösung von 2 mL Aza-cycloalkan in 2 mL Diethylener gegeben und 12 Stunden bei 20°C gerührt. Die Reaktionslösung wird im Vak. eingedampft und der Rückstand mit 10 mL Wasser versetzt. Nach dreimaliger Extraktion mit je 10 mL Essigsäureethylester werden die organischen Phasen vereinigt und über Natriumsulfat getrocknet. Das Filtrat wird im Vak. eingedampft.

### 3.2. Herstellung von mehreren speziellen Hexansäureethylestern

### 3.2.1. Beispiel 5: Herstellung von 6-(Morpholin-4-yl)-2-acetamido-2-ethoxycarbonyl-hexansäureethylester:

Ausgehend von 70 g 6-Brom-2-acetamido-2-ethoxycarbonyl-hexansäureethylester erhält man nach Umsetzung mit Morpholin und Kieselgel-Chromatographie (Dichlormethan, Methanol/Gradient: 0 auf 10% Methanol; v/v) 35 g Reinprodukt. Schmp. 58-59°C.

### 3.2.2. Beispiel 6: Herstellung von 6-(Pyrrolidin-1-yl)-2-acetamido-2ethoxycarbonyl-hexansäureethylester:

Ausgehend von 352 mg 6-Brom-2-acetamido-2-ethoxycarbonyl-hexansäureethylester erhält man nach Umsetzung mit Pyrrolidin und Kieselgel-Chromatographie (Dichlormethan, Methanol 7:3; v/v) 251 mg Reinprodukt.
Schmp. 79-81°C.

### 3.2.3. Beispiel 7: Herstellung von 6-(1-Methyl-piperazin-4-yl)-2-acetamido-2-ethoxycarbonyl-hexansäureethylester:

Ausgehend von 352 mg 6-Brom-2-acetamido-2-ethoxycarbonyl-hexansäureethylester erhält man nach Umsetzung mit 1-Methyl-piperazin und Kieselgel-Chromatographie (Dichlormethan, Methanol 7:3; v/v das Reinprodukt.

### 3.2.4. Beispiel 8: Herstellung von 6-(Piperidin-1-yl)-2-acetamido-2-ethoxycarbonyl-hexansäureethylester

Ausgehend von 352 mg 6-Brom-2-acetamido-2-ethoxycarbonyl-hexansäureethylester erhält man nach Umsetzung mit Piperidin und Kieselgel-Chromatographie (Dichlormethan. Methanol 7:3; v/v) 148 mg Reinprodukt.
Schmp. 73-75°C.

### 3.2.5. Beispiel 9: Herstellung von 6-(Imidazol)-2-acetamido-2-ethoxycarbonyl-hexansäureethylester:

Ausgehend von 352 mg 6-Brom-2-acetamido-2-ethoxycarbonyl-hexansäureethylester erhält man nach Umsetzung mit Imidazol und Kieselgel-Chromatographie (Dichlormethan, Methanol/Gradient: 0 auf 10 % Methanol; v/v) das Reinprodukt.

### 3.2.6. Beispiel 10: Herstellung von 6-(Pyrazol)-2-acetamido-2-ethoxycarbonyl-hexansäureethylester:

Ausgehend von 352 mg 6-Brom-2-acetamido-2-ethoxycarbonyl-hexansäureethylester erhält man nach Umsetzung mit Pyrazol und Kieselgel-Chromatographie (Dichlormethan, Methanol 7:3; v/v) das Reinprodukt.

### 4. Herstellung von (S)-6-(Thiomorpholin-1,1-dioxid-4-yl)-2-amino-hexansäure (Beispiel 12):

### 4.1. Herstellung von (S)-6-Amino-2-benzyloxycarbonylamino-hexansäurebenzylester (Z-Lys-OBzl)

Die Verbindung wurde nach bekanntem Verfahren hergestellt; z.B.: E. Wünsch., in: "Methoden der Organischen Chemie, Band XV/1: "Synthese von Peptiden" (Georg Thieme Verlag, 1974). B. Bezas, L. Zervas; J.Am.Chem.Soc. 83, 719 (1961).

### 4.2. (S)-6-(Thiomorpholin-1,1-dioxid-4-yl)-2-benzyloxycazbonylamino-hexansäurebenzylester:

5.6 g Z-Lys-OBzl werden im Gemisch aus 750 mL Methanol und 750 mL Dichlormethan gelöst. Nach Zugabe von 1,8 g Divinylsulfon wird 6 Stunden bei +20°C gerührt. Das Lösungsmittel wird im Vak. abdestilliert und der Rückstand chromatographisch an Kieselgel gereinigt (Gradient 0 bis 10 % Ethylacetat / tert.Butyl-methylether). Ausb.: 1,8 g (Öl).

### 4.3. (S)-6-(Thiomorpholin-1,1-dioxid-4-yl)-2-amino-hexansäure

Die Abspaltung der Schutzgruppen wird nach bekanntem Verfahren durchgeführt; z.B.: E. Wünsch, in: "Methoden der Organischen Chemie, Band XV/1: Synthese von Peptiden" (Georg Thieme Verlag, 1974).

### 5. Umwandlung der (1-Aza-cycloalk-1-yl)-2-acetamido-2-ethoxycarbonyl-pentansäure- bzw. -hexansäureethylester in die entsprechenden ungeschützten α-Aminosäuren.

Die genannten Vorläuferstufen, die entsprechend dem Herstellungsverfahren als Enantiomerengemisch vorliegen, werden nach Verfahren, die dem Chemiker wohlbekannt sind, zunächst partiell zu (1-Aza-cycloalk-1-yl)-2-acetamido2-carboxy-pentansäure- bzw. -hexansäureethylestern verseift und dann zur (1-Aza-cycloalkyl-1-yl)-2-acetamido-pentansäure bzw. hexansäure decarboxyliert. Nach enzymatischer Racemat-Trennung werden durch Totalhydrolyse die enantiomeren-reinen Aminosäuren erhalten.

### Die Verfahren wurden beispielsweise beschrieben von:

C.K. Acosta et al.; J. Chem. Research (M) 11, 914-934 (1991)
K. Folkers et al.; Int. J. Pept.Prot. Res. 24, 197-200 (1984)

### 6. Herstellung der Peptide

Die Peptide können entweder nach der Festphasen-Technik oder nach der klassischen Lösungs-Technik hergestellt werden.

Die Festphasen-Technik ist z.B. in J.M. STEWARD and J.D. YOUNG, Solid Phase Peptide Synthesis, Pierce Chem. Company, Rockford III 1984 beschrieben, die Lösungs-Technik ist z.B. in Methoden der Organischen Chemie (HOUBEN/WEYL), Bd 15 / Nr. 1 u. 2. E. WÜNSCH (Hrsg), Thieme Verlag Stuttgart, 1974 dargestellt.

Das gemeinsame Merkmal aller dieser Synthesen ist die Blockierung der α-Aminogruppe und der eventuell vorhandenen reaktiven Seitanketten-Gruppen in der Weise, daß die α-Aminogruppe selektiv freigesetzt werden kann. Diese Strategie erlaubt eine Aktivierung und selektive Reaktion der Carboxylgruppe der N-geschützten Aminosäure mit der freien α-Aminogruppe einer zweiten Aminosäure. Nach erfolgter Kupplung kann die α-Aminoschutzgruppe abgespalten und die nächste Kupplung ausgeführt werden. Im Fall der Festphasen-Synthese ist die C-terminale Carboxylgruppe am Trägerharz gebunden, bei der Lösungsmethode kann sie durch eine geeignete Gruppe geschützt sein. Bei beiden Methoden können auch statt der einzelnen Aminosäuren geeignete Peptidfragmente verknüpft werden. Man erhält nach beiden Methoden ein Polypeptid mit geschützten oder teilgeschützten Seitenkettenfunktionen. Nach Abspaltung der Schutzgruppen kann durch HPLC das gewünschte Peptid rein erhalten werden.

### Beispiel 13 bis 15

Beispielhaft für die Peptid-Verbindung werden drei Decapeptide angeführt, deren Herstellung zuvor beschrieben ist. Die Eigenschaften der drei Decapeptide sind tabellarisch aufgeführt. Die Legende für die Abkürzungen befindet sich im Anschluß am Beispiel 15.

### 6.1. Beispiel 13

Ac-D-Nal-D-Cpa-D-Pal-Ser-Tyr-D-Cit-Leu-Ahx(Mor)-Pro-D-Ala-NH₂

| | D-Nal | D-Cpa | D-Pal | Ser | Tyr | D-Cit | Leu | Ahx(Mor) | Pro | D-Ala |
|---|---|---|---|---|---|---|---|---|---|---|
| ASA ber. | 1.00 | 1.00 | 1.00 | 1.00 | 1.00 | 1.00 | 1.00 | 1.00 | 1.00 | 1.00 |
| ASA^{a)} gef. | 1.03 | 0.98 | 1.01 | 0.95 | 0.97 | 1.03 | 1.01 | 0.96 | 1.00 | 0.97 |
| RAC^{b)} | <1 | 1.2 | 2.5 | <1 | <1 | ^{c)} | <1 | ^{c)} | 1.1 | 1.3 |

FAB-MS Molekülpeak m/e 1472.6 (+H). Berechnete Molmasse 1473.1

Die Peptid-Verbindung des Beispiels 13 ist die bevorzugte Ausführungsform.

### 6.2. Beispiel 14

Ac-D-Nal-D-Cpa-D-Pal-Ser-Tyr-D-Ahx(Mor)-Leu-Arg-Pro-D-Ala-NH₂

| | D-Nal | D-Cpa | D-Pal | Ser | Tyr | D-Ahx(Mor) | Leu | Arg | Pro | D-Ala |
|---|---|---|---|---|---|---|---|---|---|---|
| ASA ber. | 1.00 | 1.00 | 1.00 | 1.00 | 1.00 | 1.00 | 1.00 | 1.00 | 1.00 | 1.00 |
| ASA^{a)} gef. | 1.05 | 1.02 | 1.03 | 0.95 | 0.96 | 0.97 | 1.00 | 0.96 | 0.98 | 0.95 |
| RAC^{b)} | <1 | 1.6 | 3.3 | <1 | 0.8 | 1.0 | <1 | <1 | <1 | 1.3 |

FAB-MS Molekülpeak m/e 1471.9 (+H). Berechnete Molmasse 1472.2

### 6.3. Beispiel 15

Ac-D-Nal-D-Cpa-D-Pal-Ser-Tyr-D-Ahx(Mor)-Leu-Ahx(Mor)-Pro-D-Ala-NH₂

| | D-Nal | D-Cpa | D-Pal | Ser | Tyr | D-Ahx(Mor) | Leu | Pro | D-Ala |
|---|---|---|---|---|---|---|---|---|---|
| ASA ber. | 1.00 | 1.00 | 1.00 | 1.00 | 1.00 | 2.00 | 1.00 | 1.00 | 1.00 |
| ASA^{a)} gef. | 1.05 | 1.01 | 1.01 | 0.95 | 0.95 | 1.93 | 0.97 | 0.98 | 0.96 |
| RAC^{b)} | 1.6 | 1.5 | 2.6 | <1 | 0.8 | ^{d)} | <1 | <1 | 1.1 |

FAB-MS Molekülpeak m/e 1513.8 (+H). Berechnete Molmasse 1514.2
a) ASA = Aminosäureanalyse des Hydrolysats. Hydrolysebedingungen: 6M HCl-Lösung, 110°C, 24 Stunden.
b) RAC = Anteil des unerwünschten Enantiomeren in Prozent. Bestimmung nach H. Frank, G.J. Nicholson und E. Bayer, J. Chromatogr.Science 15, 174 (1977).
c) Die Bestimmung ist nicht möglich, da die untersuchten Cit- und Ahx(Mor)-Derivate die gleichen gaschromatographischen Retentionszeiten aufweisen
d) Das Verhältnis von D-Ahx(Mor):L-Ahx(Mor) beträgt 1:1.

## Patentansprüche

1. Peptid-Verbindungen der Formel I, 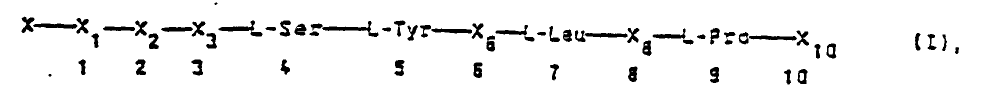 worin
X für eine Naphthoyl-, Naphthylacetyl-, Naphthylpropionyl-, Benzoylgruppe oder eine Acylgruppe mit 1-7 Kohlenstoff-Atomen steht,
X₁ für D-(1)-Nal, D-(2)-Nal, D-Phe, D-(4-Y)-Phe, D-(3)-Qal oder eine Direktbindung steht,
wobei Y eine F-, Br- oder Cl-Gruppe ist,
X₂ für D-Phe, D-(4-Y)-Phe oder eine Direktbindung steht,
wobei Y die zuvor genannte Bedeutung besitzt,
X₃ für D-Trp, D-Phe, D-(4-Y)-Phe, D-(3)-Pal, D-(2)-Nal oder eine Direktbindung steht,
wobei Y die bereits für X₁ genannte Bedeutung besitzt,
X₆ für D-Cit, D-Hci, D-Orn, D-Lys oder D-Neu steht,
X₈ für L-Orn, L-Arg, L-Lys oder L-Neu steht, wobei wenigstens einer der Reste X₆ und X₈ ein Neu ist,
und
X₁₀ für D-Ala-NH₂, Gly-NH₂, Azaglycin, -NHEt oder -NH(CO)NH₂ steht,
wobei Neu für einen Rest der Formel IX oder IV 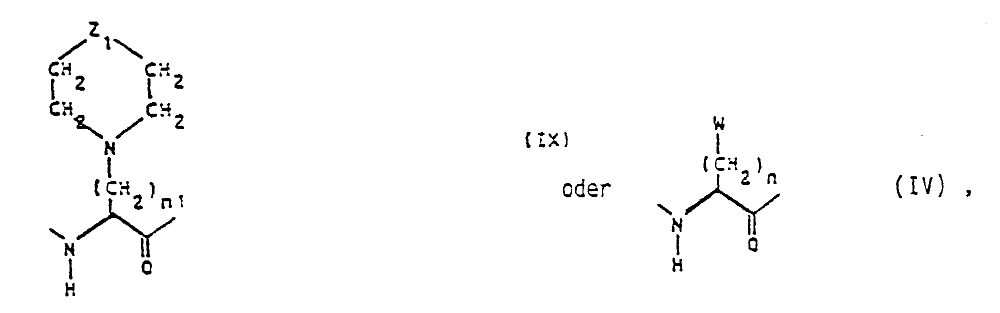 steht, worin Z₁ für eine Gruppe 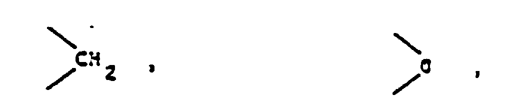 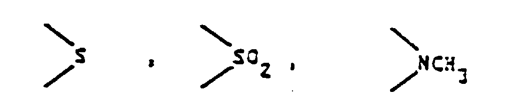 oder eine Direktbindung,
n für 1 bis 8,
n₁ für 3 bis 6 Stehen und
W einen der Reste 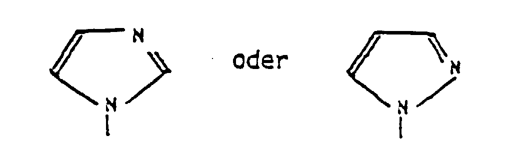 bedeutet.

2. Peptid-Verbindungen nach Anspruch 1, bei denen Neu für eine Gruppe der Formel X steht, 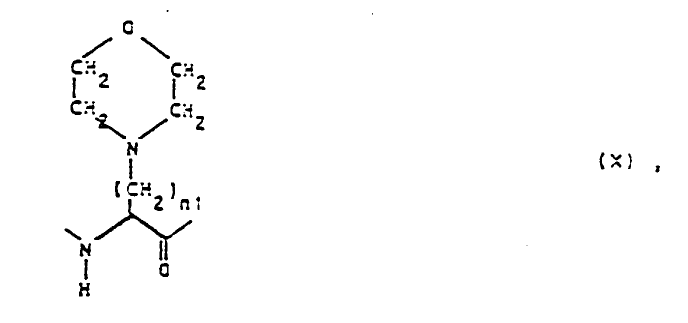 worin n₁ für 3 bis 6 steht.

3. Peptid-Verbindungen nach Anspruch 2, bei denen Neu für den Rest 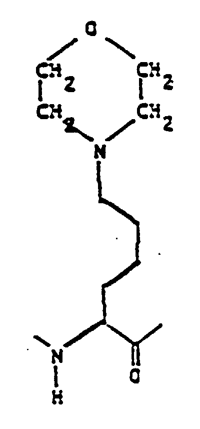 steht.

4. Peptid-Verbindungen nach Anspruch 3, bei denen Neu für X₆ oder X₈ steht.

5. Peptid-Verbindungen nach Anspruch 1, wobei Neu für die Formel XIII steht.

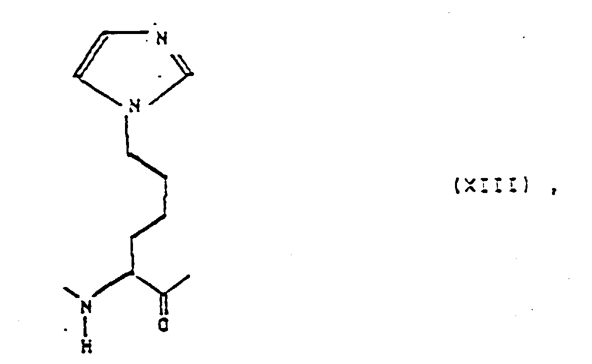

6. Peptid-Verbindungen nach einem der Ansprüche 1 bis 5, worin
X für eine Ethancyl-Gruppe,
X₁ für D-Nal,
X₂ für D-Cpa,
X₃ für D-Pal und
X₁₀ für D-Ala-NH₂ steht.

7. Peptid-Verbindung nach einem der Ansprüche 1 bis 4, die Ac-D-Nal-D-Cpa-D-Pal-Ser-Tyr-D-Cit-Leu-Ahx (Mor)-Pro-D-Ala-NH₂ ist.

8. Arzneimittel enthaltend eine oder mehrere Verbindungen der Formel I, gemäß den Ansprüchen 1 - 7 und übliche Hilfs- und Trägerstoffe.

## Claims

1. Peptide compounds of formula I 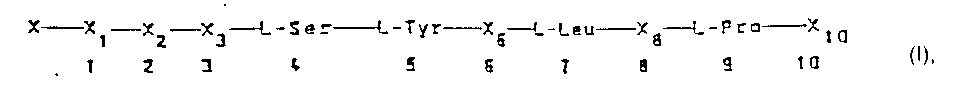 wherein
X represents a naphthoyl, naphthylacetyl, naphthylpropionyl or benzoyl group or an acyl group having from 1 to 7 carbon atoms,
X₁ represents D-(1)-Nal, D-(2)-Nal, D-Phe, D-(4-Y)-Phe, D-(3)-Qal or a direct bond, wherein Y is an F, Br or Cl group,
X₂ represents D-Phe, D-(4-Y)-Phe or a direct bond,
wherein Y has the meanings mentioned above,
X₃ represents D-Trp, D-Phe, D-(4-Y)-Phe, D-(3)-Pal, D-(2)-Nal or a direct bond, wherein Y has the meanings already mentioned for X₁,
X₆ represents D-Cit, D-Hci, D-Orn, D-Lys or D-Neu,
X₈ represents L-Orn, L-Arg, L-Lys or L-Neu, at least one of the residues X₆ and X₈ being a Neu,
and
X₁₀ represents D-Ala-NH₂, Gly-NH_{2,} azaglycine, -NHEt or -NH(CO)NH₂,
wherein Neu represents a residue of formula IX or IV 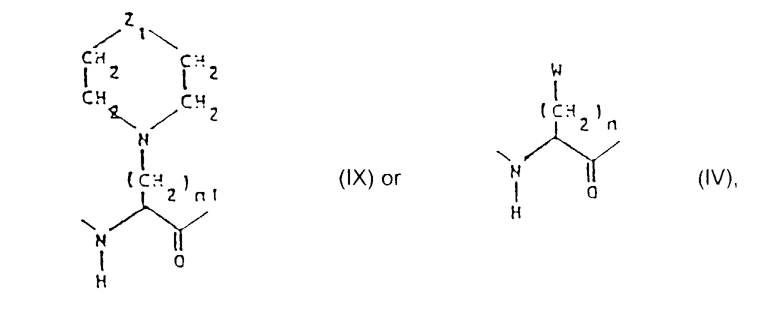 wherein Z₁ represents a group 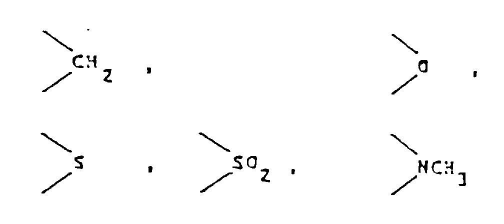 or a direct bond,
n represents 1 to 8,
n₁ represents 3 to 6 and
W represents one of the radicals

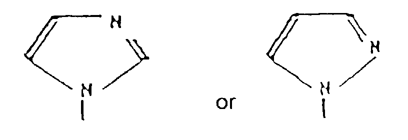

2. Peptide compounds according to claim 1, in which Neu represents a group of formula X 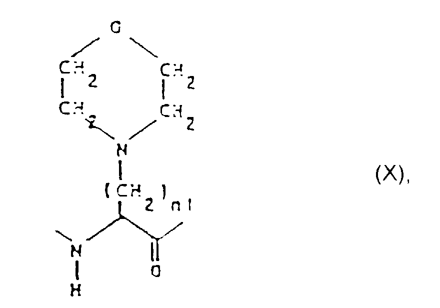 wherein n₁ is from 3 to 6.

3. Peptide compounds according to claim 2, in which Neu represents the residue

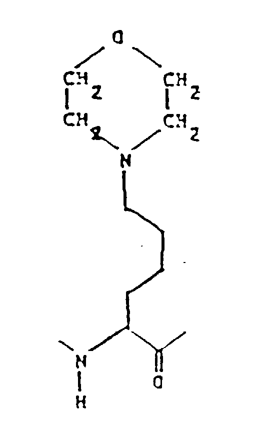

4. Peptide compounds according to claim 3, in which Neu represents X₆ or X₈.

5. Peptide compounds according to claim 1, in which Neu represents the formula XIII

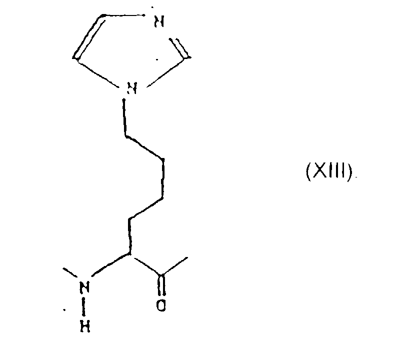

6. Peptide compounds according to any one of claims 1 to 5, wherein X represents an ethanoyl group,
X₁ represent D-Nal,
X₂ represents D-Cpa,
X₃ represents D-Pal and
X₁₀ represents D-Ala-NH₂.

7. Peptide compound according to any one of claims 1 to 4, which is
Ac-D-Nal-D-Cpa-D-Pal-Ser-Tyr-D-Cit-Leu-Ahx(Mor)-Pro-D-Ala-NH₂.

8. Medicament comprising one or more compounds of formula I according to claims 1 to 7 and customary adjuvants and carriers.

## Revendications

1. Composés peptidiques de la formule I 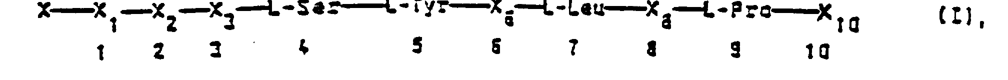 dans laquelle
X représente un groupe naphtoyle, naphtylacétyle, naphtylpropionyle, benzoyle ou un groupe acyle comportant 1 à 7 atomes de carbone,
X₁ représente D-(1)-Nal, D-(2)-Nal, D-Phe, D-(4-Y)-Phe, D-(3)-Qal ou une liaison directe,
Y étant un groupe F, Br ou Cl,
X₂ représente D-Phe. D-(4-Y)-Phe ou une liaison directe.
Y ayant la signification donnée ci-dessus,
X₃ représente D-Trp, D-Phe, D-(4-Y)-Phe, D-(3)-Pal, D-(2)-Nal ou une liaison directe,
Y ayant la signification déjà donnée pour X₁,
X₆ représente D-Cit. D-Hci, D-Orn, D-Lys ou D-Neu,
X₈ représente L-Orn. L-Arg, L-Lys ou L-Neu, au moins un des radicaux X₆ et X₈ étant un Neu,
et
X₁₀ représente D-Ala-NH₂, Gly-NH₂, azaglycine. -NHEt ou -NH(CO)NH₂,
Neu représentant un radical de la formule IX ou IV 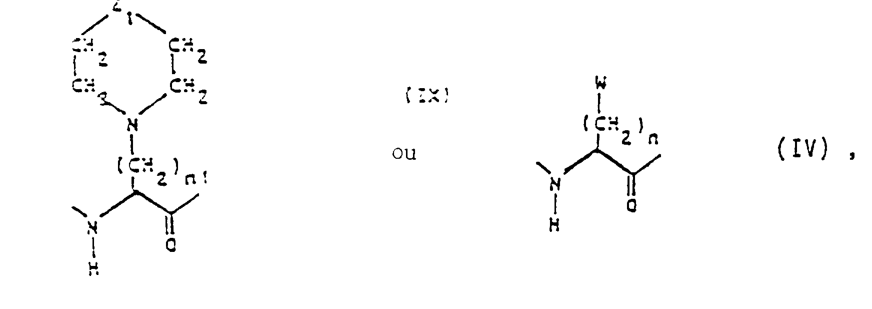 où Z₁ représente un groupe 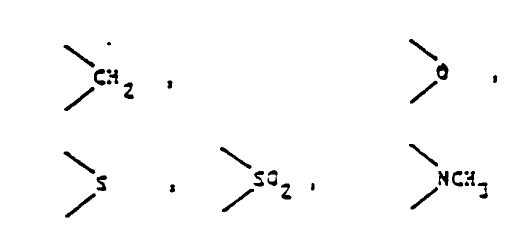 ou une liaison directe,
n représente 1 à 8,
n₁ représente 3 à 6, et
W représente un des radicaux

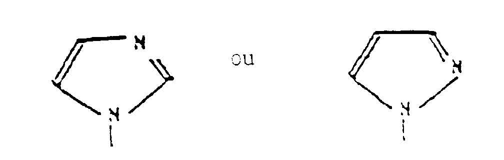

2. Composés peptidiques suivant la revendication 1, dans lesquels Neu représente un groupe de la formule X 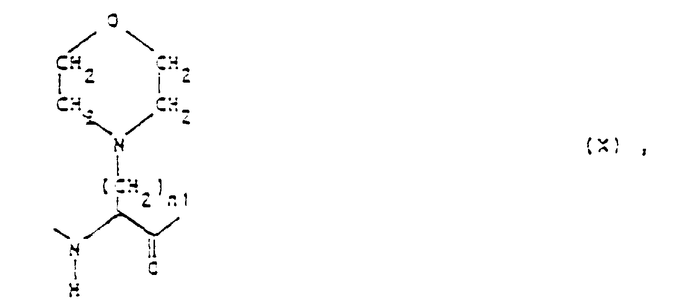 où n₁ représente 3 à 6.

3. Composés peptidiques suivant la revendication 2, dans lesquels Neu représente le radical

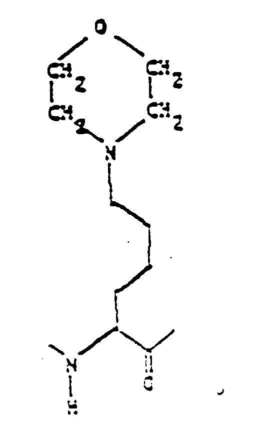

4. Composés peptidiques suivant la revendication 3, dans lesquels X₆ ou X₈ représente Neu.

5. Composés peptidiques suivant la revendication 1, caractérisés en ce que Neu répond à la formule XIII

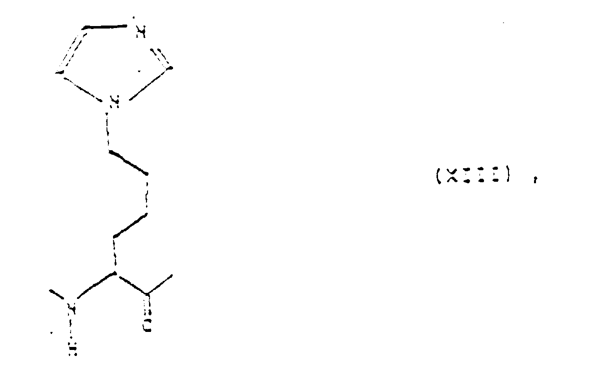

6. Composés peptidiques suivant l'une des revendications 1 à 5, caractérisés en ce que
X représente un groupe éthanoyle,
X₁ représente D-Nal,
X₂ représente D-Cpa,
X₃ représente D-Pal,
et
X₁₀ représente D-Ala-NH₂.

7. Composé peptidique suivant l'une des revendications 1 à 4, qui est du
Ac-D-Nal-D-Cpa-D-Pal-Ser-Tyr-D-Cit-Leu-Ahx (Mor)-Pro-D-Ala-NH₂.

8. Médicament contenant un ou plusieurs des composés de la formule I, selon l'une des revendications 1 à 7, et des adjuvants et supports courants.
